# EUROPÄISCHE PATENTANMELDUNG

(11) **EP 3 444 193 A1**
(43) Veröffentlichungstag der Anmeldung: **20.02.2019**
(21) Anmeldenummer: 17186159.4
(22) Anmeldetag: 14.08.2017
(51) Int. Cl.: B65B 55/10, A61L 2/20

(54) **DEKONTAMINATIONSVORRICHTUNG, ISOLATORSYSTEM SOWIE BETRIEBSVERFAHREN**

(71) Anmelder: Metall + Plastic GmbH, 78315 Radolfzell (DE)
(72) Erfinder: Kleinmann, Stefan, 78315 Radolfzell (DE)
(74) Vertreter: Patentanwälte Behrmann Wagner PartG mbB

(57) **Zusammenfassung**

Die Erfindung betrifft eine Dekontaminationsvorrichtung zum Dekontaminieren von Verbrauchsgüterumverpackungen mit darin befindlichen, bevorzugt als Primärverpackungen ausgebildeten, vorsterilisierten Verbrauchsgütern vor dem Zuführen der Verbrauchsgüter in einen Isolator, umfassend eine Dekontaminationskammer (2), aufweisend Beaufschlagungsmittel (3) zum Beaufschlagen der Verbrauchsgüterumverpackungen mit einem Dekontaminationsmittel, insbesondere einem Dekontaminationsmitteldampf oder -aerosol, wobei die Dekontaminationskammer (2) Transportmittel (6) zum gleichzeitigen Transportieren einer Mehrzahl von gleichzeitig zu dekontaminierenden Verbrauchsgüterumverpackungen während der Dekontamination von einem Einlass (4) zu einem Auslass (5) der Dekontaminationskammer (2) umfasst und dass der Dekontaminationskammer (2) eine Beladeschleuse (7) vorgeordnet ist, die mit zu dekontaminierenden Verbrauchsgüterumverpackungen beladbar ist, und dass der Dekontaminationskammer (2) eine Entpackungsschleuse (14) nachgeordnet ist, in der die sterilen Verbrauchsgüter vor dem Weitertransport in den Isolator aus der zuvor dekontaminierten Verbrauchsgüterumverpackung, insbesondere automatisiert, entpackbar sind, und dass der Beladeschleuse (7) und der Entpackungsschleuse (14) Mittel zum Absenken der Dekontaminationsmittelkonzentration (8) in der Beladeschleuse (7) bzw. der Entpackungsschleuse (14) in einem von der Dekontaminationskammer (2) abgekoppelten Betriebszustand zugeordnet sind.

## Beschreibung

Die Erfindung betrifft eine Dekontaminationsvorrichtung gemäß dem Oberbegriff des Anspruchs 1 zum Dekontaminieren von Verbrauchsgüterverpackungen mit darin befindlichen, bevorzugt als Primärverpackungen ausgebildeten, vorsterilisierten (d.h. sterilen) Verbrauchsgütern vor dem Zuführen der Verbrauchsgüter in einen Isolator, umfassend eine Dekontaminationskammer, aufweisend Beaufschlagungsmittel zum Beaufschlagen der Verbrauchsgüterumverpackungen mit einem Dekontaminationsmittel, insbesondere einem Dekontaminationsmitteldampf oder Aerosol, ganz besonders bevorzugt mit einem Wasserstoffperoxiddampf oder -aerosol.

Ferner betrifft die Erfindung ein Isolatorsystem, insbesondere für pharmatechnische Anwendungen, gemäß Anspruch 8, umfassend einen Isolator sowie eine diesen vorgeordnete, erfindungsgemäße Dekontaminationsvorrichtung.

Darüber hinaus betrifft die Erfindung ein Verfahren gemäß dem Oberbegriff des Anspruchs 9 zum Betreiben einer erfindungsgemäßen Dekontaminationsvorrichtung und/oder eines erfindungsgemäßen Isolatorsystems.

Es ist bekannt, Verbrauchsgüter, wie Arzneimittelverpackungen bzw. -behälter und/oder deren Umverpackungen vor der Zuführung der Verbrauchsgüter in ein Isolator zu dekontaminieren, wozu die Verbrauchsgüter bzw. Verbrauchsgüterumverpackungen in einer Dekontaminationskammer mit Dekontaminationsmittel, meist in der Form von Wasserstoffperoxiddampf beaufschlagt und danach steril den Isolator zugeführt werden. Für den Fall der Dekontamination von Umverpackungen werden die Verbrauchsgüter vor der Zuführung zum Isolator aus der innen sterilen und wie zuvor beschrieben dekontaminierten Umverpackung ausgepackt. Bei bekannten Dekontaminationsvorrichtungen wird als nachteilig empfunden, dass die Zeitspanne zwischen Beschickung der Dekontaminationskammer und Zuführen in den Isolator lang ist. Dies ist insbesondere darauf zurückzuführen, dass die Dekontaminationskammer zur Vermeidung einer Belastung der Bedienperson zum Beladezeitpunkt frei ist von Dekontaminationsmittel und nach dem Schließen der Dekontaminationskammer zunächst die Dekontaminationsmittelkonzentra-tion in der Dekontaminationskammer (wieder) angehoben, während der Dekontaminationszeit aufrechterhalten und danach vor dem Öffnen einer Verbindung zum Isolator wieder reduziert werden muss, um eine Leckage von Dekontaminationsmittel in den Isolator hinein zu vermeiden, da das Dekontaminationsmittel, meist Wasserstoffperoxid, negative Einflüsse auf die in dem Isolator befindlichen medizinischen Wirkstoffe haben kann.

Ausgehend von dem vorgenannten Stand der Technik liegt der Erfindung die Aufgabe zugrunde, eine verbesserte Dekontaminationsvorrichtung, ein verbessertes Isolatorsystem sowie ein entsprechendes, verbessertes Betriebsverfahren anzugeben, mit denen eine schnellere, bevorzugte quasikontinuierliche Beschickung des Isolators mit Verbrauchsgütern möglich ist. Insbesondere soll die Aufenthaltszeit der Verbrauchsgüterumverpackungen in einer Dekontaminationskammer der Dekontaminationsvorrichtung verglichen mit dem Stand der Technik reduziert werden.

Diese Aufgabe wird hinsichtlich der Dekontaminationsvorrichtung mit den Merkmalen des Anspruchs 1 gelöst, d.h. bei einer gattungsgemäßen Dekontaminationsvorrichtung dadurch, dass die Dekontaminationskammer Transportmittel zum gleichzeitigen, insbesondere hintereinander, Transportieren einer Mehrzahl von gleichzeitig zu dekontaminierenden (aus unterschiedlichen Belade- bzw. Beschichtungsvorgängen der Dekontaminationskammer stammenden) Verbrauchsgüterverpackungen während der Dekontamination von einem Einlass zu einem (in einer Transportrichtung von dem Einlass beabstandeten) Auslass der Dekontaminationskammer umfasst und dass der Dekontaminationskammer eine Beladeschleuse vorgeordnet ist, die mit zu dekontaminierenden Verbrauchsgüterumverpackungen beladbar ist, und dass der Dekontaminationskammer eine Entpackungsschleuse nachgeordnet ist, in der die sterilen Verbrauchsgüter vor dem Weitertransport in den Isolator aus der zuvor dekontaminierten Verbrauchsgüterumverpackung, insbesondere automatisiert, entpackbar sind, und dass der Beladeschleuse und der Entpackungsschleuse Mittel zum Absenken der Dekontaminationsmittelkonzentration (in der jeweiligen Schleuse) in einem von der Dekontaminationskammer abgekoppelten Betriebszustand zugeordnet sind. Unter dem von der Dekontaminationskammer abgekoppelten Betriebszustand wird ein Betriebszustand verstanden, bei der eine Verbindung zwischen der Dekontaminationskammer zu der jeweiligen Schleuse, insbesondere eine entsprechende Schleusentür verschlossen ist, sodass eine Absenkung der Dekontaminationsmittelkonzentration in der jeweiligen Schleuse den zwischenzeitlich weiterlaufenden Dekontaminationsprozess in der Dekontaminationskammer nicht negativ beeinflusst.

Hinsichtlich des Isolatorsystems wird die Aufgabe mit den Merkmalen des Anspruchs 8 gelöst, wobei die aus den dekontaminierten Verbrauchsgüterverpackungen (in der Entpackungsschleuse) entpackten Verbrauchsgüter aus der Dekontaminationsvorrichtung, insbesondere unmittelbar aus der Entpackungsschleuse, dem Isolator, insbesondere einem Manipulatorraum des Isolators, steril zuführbar sind.

Hinsichtlich des Verfahrens wird die Aufgabe mit den Merkmalen des Anspruchs 9 gelöst, d.h. bei einem gattungsgemäßen Verfahren dadurch, dass gleichzeitig eine Mehrzahl von Verbrauchsgüterumverpackungen mit denen die Dekontaminationskammer nacheinander beladen wurde in der Dekontaminationskammer dekontaminiert werden und während der Dekontamination mit den Transportmitteln von dem Einlass zu dem (in der Transportrichtung von dem Einlass beabstandeten) Auslass der Dekontaminationskammer transportiert werden, und dass die der Dekontaminationskammer vorgeordnete Beladeschleuse mit zu dekontaminierenden Verbrauchsgüterverpackungen beladen wird, die aus der Beladeschleuse (einzeln oder in Gruppen), bevorzugt nacheinander der Dekontaminationskammer zugeführt werden und dass in der der Dekontaminationskammer nachgeordneten Entpackungsschleuse die sterilen (vorsterilisierten) Verbrauchsgüter, insbesondere Primärverpackungen für Pharmazeutika, vor dem Weitertransport in den Isolator aus den zuvor dekontaminierten Verbrauchsgüterverpackungen, insbesondere automatisiert, entpackt werden, und dass in der Beladeschleuse vor dem Beladen mit einer Verbrauchsgüterumverpackung die Dekontaminationsmittelkonzentration, insbesondere durch Verdrängen von Dekontaminationsmittel mit Frischluft und/oder durch Fördern von Schleusenraumluft im Umluftbetrieb durch einen das Dekontaminationsmittel abbauenden Katalysator, abgesenkt wird, und dass in der Entpackungsschleuse vor dem Überführen der entpackten Verbrauchsgüter in den Isolator die Dekontaminationsmittelkonzentration (wie zuvor im Zusammenhang mit der Beladeschleuse beschrieben) abgesenkt wird.

Vorteilhafte Weiterbildungen der Erfindung sind in den Unteransprüchen angegeben.

In den Rahmen der Erfindung fallen sämtliche Kombinationen aus zumindest zwei von der in der Beschreibung, den Ansprüchen und/oder Figuren offenbarten Merkmalen.

Zur Vermeidung von Wiederholungen sollen vorrichtungsgemäß offenbarte Merkmale auch als verfahrensgemäß offenbart gelten und beanspruchbar sein. Ebenso sollen verfahrensgemäß offenbarte Merkmale auch als vorrichtungsgemäß offenbart gelten und beanspruchbar sein.

Der Erfindung liegt der Gedanke zugrunde, die Dekontaminationskammer als, bevorzugt langgestreckten Tunnel auszubilden, in welchem die Verbrauchsgüterumverpackungen während der Dekontamination, d. h. während der Beaufschlagung mit Dekontaminationsmittel, insbesondere Wasserstoffperoxid, von einem Einlass der Dekontaminationskammer zu einem Auslass der Dekontaminationskammer (in Richtung Isolator) transportiert werden, wobei die Dekontaminationskammer eingerahmt ist bzw. angeordnet ist zwischen zwei Schleusen, nämlich einer vorderseitigen Beladeschleuse und einer rückwärtigen Entpackungsschleuse. Beiden Schleusen sind Mittel zum Absenken der Dekontaminationsmittelkonzentration zugeordnet, um im Falle der Beladeschleuse eine Gefährdung von Bedienpersonal und der Umwelt durch Dekontaminationsmittelleckage zu vermeiden und im Falle der Entpackungsschleuse eine Leckage von Dekontaminationsmittel in den Isolator zu vermeiden. Die Beladeschleuse wird mit zu dekontaminierten Verbrauchsgüterumverpackungen beladen, wobei vor dem Öffnen einer Einlass- bzw. Beladetür der Beladeschleuse und bei geschlossener Verbindung zur Dekontaminationskammer die Dekontaminationsmittelkonzentration in der Beladeschleuse abgesenkt wird, woraufhin eine Einlass- bzw. Beladetür der Beladeschleuse geöffnet und die Beladeschleuse mit einer einzigen oder mehreren Verbrauchsgüterumverpackung (mit darin befindlichen, sterilen Verbrauchsgütern) beladen wird. Daraufhin wird die Einlass- bzw. Beladetür der Beladeschleuse geschlossen und die Verbindung zwischen Beladeschleuse und Dekontaminationskammer geöffnet, d. h. die Beladeschleuse wird an die Dekontaminationskammer angekoppelt, um die einzige oder die mehreren Verbrauchsgüterumverpackungen in die Dekontaminationskammer zu überführen, in welcher sie mittels der Transportmittel in Richtung des Auslasses transportiert wird/werden unter gleichzeitiger Beaufschlagung mit Dekontaminationsmittel. Daraufhin wird die Beladeschleuse wieder von der Dekontaminationskammer abgekoppelt und die Dekontaminationsmittelkonzentration, die aus dem Ankoppeln der Beladeschleuse an die Dekontaminationskammer bzw. der darauf auftretenden Leckage resultiert wieder abgesenkt wird, um die Beladeschleuse erneut beladen zu können und nach dem Schließen der Einlass- bzw. Beladetür die mindestens eine Verbrauchsgüterverpackung dem Dekontaminationstunnel (Dekontaminationskammer) zuzuführen, wobei in dieser hierfür entsprechend Platz bzw. Raum geschaffen wurde, da die in dem vorhergehenden Beladevorgang zugeführte, mindestens eine Verbrauchsgüterumverpackung weiter in Richtung Auslass transportiert wurde (aber sich immer noch in der Dekontaminationskammer befindet). Auslassseitig ist dem Dekontaminationstunnel, wie erwähnt, die Entpackungsschleuse zugeordnet, in der die Verbrauchsgüter (insbesondere Pharmazeutikaprimärverpackungen) vor dem Zuführen in den Isolator entweder manuell oder bevorzugt durch eine entsprechende Robotik automatisiert entpackt werden. Jedenfalls wird die Dekontaminationsmittelkonzentration, die aus einer Leckage während des Überführens der mindestens einen dekontaminierten Verbrauchsgüterumverpackung aus der Dekontaminationskammer in die Entpackungsschleuse resultiert, abgesenkt und zwar bei geschlossener Verbindung zwischen Entpackungsschleuse und Dekontaminationskammer, d.h. bei von der Dekontaminationskammer abgekoppelter/entkoppelter Entpackungsschleuse noch vor dem Öffnen der Verbindung zwischen Entpackungsschleuse und Isolator, um somit eine Verschleppung von Dekontaminationsmittel in den Isolator zu vermeiden.

Im Ergebnis wird durch die erfindungsgemäße Dekontaminationsvorrichtung bzw. das erfindungsgemäße Betriebsverfahren eine quasikontinuierliche Beschickung des Isolators mit Verbrauchsgütern, insbesondere Primärverpackungen ermöglicht, da aufgrund der langgestreckten bzw. tunnelartigen Ausgestaltung der Dekontaminationskammer gleichzeitig nacheinander der Dekontaminationskammer zugeführte und vom Einlass zum Auslass transportierte Verbrauchsgüterumverpackungen während des Transportes dekontaminiert werden können bzw. dekontaminiert werden. Nacheinander zugeführte Verbrauchsgüterumverpackungen bedeutet, dass zwischen nacheinander zugeführten Verbrauchsgüterverpackungen ein vollständiger bzw. neuer Beladevorgang der Beladeschleuse liegt, welcher es erforderlich macht, nach Beschickung der Dekontaminationskammer mit einer oder mehreren Verbrauchsgüterumverpackungen aus der Beladeschleuse für einen erneuten Beladevorgang die Beladeschleuse von der Dekontaminationskammer abzukoppeln, die Dekontaminationsmittelkonzentration in der Beladeschleuse abzusenken, daraufhin die Beladeschleuse mit einer oder mehreren Verbrauchsgüterverpackungen (erneut) zu beladen und daraufhin diese eine oder die mehreren Verbrauchsgüterverpackungen der Dekontaminationskammer zuzuführen, wobei die mindestens eine zuvor bzw. bei dem vorhergehenden Beladevorgang zugeführte Verbrauchsgüterumverpackung sich noch innerhalb der Dekontaminationskammer auf dem Weg zum Auslass befindet. Bevorzugt ist die Dekontaminationskammer so bemessen, dass diese Raum hat für Verbrauchsgüterumverpackungen aus mindestens zwei, bevorzugt mehr als zwei, ganz besonders bevorzugt mehr als fünf Beladevorgängen.

Die erfindungsgemäße Dekontaminationsvorrichtung ermöglicht einen Betrieb, bei dem die Dekontaminationsmittelkonzentration in der Dekontaminationskammer zum Beladen und Entladen mit bzw. von Verbrauchsgüterumverpackungen nicht aktiv abgesenkt werden muss, da die Dekontaminationskammer zwischen zwei Schleusen angeordnet ist. Die einzige geringfügige Absenkung kann dabei dann aus dem Öffnen der Verbindung zur jeweiligen Schleuse zum Zu- oder Abführen von zu dekontaminierenden oder bereits dekontaminierten Verbrauchsgüterumverpackungen resultieren. Hieraus resultiert der Vorteil, dass die Dekontaminationsmittelkonzentration nach einer Produktzuführung nicht erst wieder von Null aufgebaut werden muss und nicht auf Null reduziert werden muss, bevor eine dekontaminierte Verbrauchsgüterumverpackung entnommen wird. Anders ausgedrückt kann somit auf die langen Rampen zum aktiven Aufbau und Abbau der Dekontaminationsmittelkonzentration in der Dekontaminationskammer nach bzw. vor einem Beladevorgang verzichtet werden, wodurch erhebliche Zeit eingespart wird.

In Weiterbildung der Erfindung ist mit Vorteil vorgesehen, dass die Baulänge der Dekontaminationskammer, d. h. des Dekontaminationstunnels so bemessen und auf eine Fördergeschwindigkeit (Transportgeschwindigkeit) der Transportmittel abgestimmt ist, dass die Verbrauchsgüterverpackungen (jeweils) auf ihrem Weg vom Einlass zum Auslass der Dekontaminationskammer, bevorzugt im Rahmen einer kontinuierlichen oder intermittierenden Förderbewegung, mindestens 5 min, bevorzugt zwischen 10min und 60min, weiter bevorzugt zwischen 10 min und 30 min, ganz besonders bevorzugt zwischen 15 min und 25 min in der mit Dekontaminationsmittel beaufschlagten Dekontaminationskammer verweilen. Ganz wesentlich ist, dass die Dekontaminationskammer so bemessen und auf die Fördergeschwindigkeit der Transportmittel abgestimmt bzw. so beladen wird, dass gleichzeitig in unterschiedlichen Belade- bzw. Beschickungsvorgängen der Dekontaminationskammer zugeführte Verbrauchsgüterumverpackungen in der Dekontaminationskammer dekontaminiert und vom Einlass zum Auslass transportiert werden, wobei die nacheinander der Dekontaminationskammer zugeführten Verbrauchsgüterverpackungen jeweils (überlappend) die notwendige Dekontaminationszeit in der Dekontaminationskammer verweilen.

Besonders zweckmäßig ist es, wenn die Verbrauchsgüterumverpackungen zwischen Einlass und Auslass der Dekontaminationskammer einen Förder- bzw. Transportweg aus einem Wertebereich zwischen 1,0 m und 15,0 m, ganz besonders bevorzugt zwischen 2,0 m und 10,0 m, besonders bevorzugt zwischen 3,0 m und 8,0 m zurücklegen.

Als besonders zweckmäßig hat es sich herausgestellt, wenn in der Beladeschleuse und/oder der Entpackungsschleuse Fördermittel für die Verbrauchsgüterumverpackungen vorgesehen sind, um diese im Falle der Beladeschleuse in die Dekontaminationskammer zu transportieren und an deren Transportmittel zu übergeben bzw. um im Falle der Entpackungsschleuse Verbrauchsgüterumverpackungen aus der Dekontaminationskammer zu übernehmen und bevorzugt die daraus entpackten Verbrauchsgüter in den Isolator zu überführen. Die Fördermittel, ebenso wie die Transportmittel der Dekontaminationskammer können dabei beispielsweise jeweils mindestens ein umlaufendes Endlosband und/oder Förderrollen oder dergleichen Transporteinrichtungen umfassen.

Die Beladeschleuse und die Entpackungsschleuse umfassen jeweils mindestens eine Einlasstür als auch eine Auslasstür, wobei die Einlasstür der Beladeschleuse eine Verbindung zur Umgebung öffnet oder schließt, um die Beladeschleuse mit Verpackungsgüterumverpackungen beladen zu können. Die Auslasstür der Beladeschleuse dient bevorzugt zum Koppeln und Entkoppeln, d.h. zum Verbinden und Unterbrechen der Verbindung der Beladeschleuse zur Dekontaminationskammer. Die Dekontaminationsmittelkonzentration in der Beladeschleuse wird bei geschlossener Einlasstür und geschlossener Auslasstür abgesenkt. Die Einlasstür der Entpackungsschleuse ermöglicht eine Verbindung und Unterbrechung der Verbindung, d.h. ein Koppeln und Entkoppeln der Entpackungsschleuse von der Dekontaminationskammer, während die Auslasstür bevorzugt eine Verbindung zum Isolator öffnet oder schließt. Selbstverständlich kann ein- und auslassseitig der Schleusen jeweils mehr als eine Tür vorgesehen sein.

Im Hinblick auf die konkrete Ausgestaltung der Mittel zum Absenken der Dekontaminationsmittelkonzentration in der Beladeschleuse und der Entpackungsschleuse gibt es unterschiedliche Möglichkeiten. Ganz besonders bevorzugt ist eine Ausführungsform dieser Absenkmittel, bei der diese Umlufterzeugermittel, insbesondere in Form eines Gebläses umfassen, mit denen ein Umluftvolumenstrom der jeweiligen Schleusenraumluft durch Katalysatormittel zum Abbauen des Dekontaminationsmittels erzeugbar ist. Ferner umfassen die Absenkmittel dann derartige Katalysatormittel. Die, beispielsweise palladiumhaltigen, Katalysatormittel sind dabei bevorzugt ausgebildet, dass diese das Dekontaminationsmittel, insbesondere Wasserstoffperoxid, in seine Bestandteile, im Falle von Wasserstoffperoxid bevorzugt in Wasser und Sauerstoff zerlegen. Zusätzlich oder alternativ können die Absenkmittel Frischluftversorgungsmittel zum Verdrängen von dekontaminationsmittelhaltiger Raumluft aus dem jeweiligen Schleusenraum, insbesondere in die Atmosphäre, ganz besonders bevorzugt über einen Katalysator zum Abbau des Dekontaminationsmittels umfassen. Grundsätzlich ist es möglich, dass auch die Dekontaminationskammer (separate bzw. eigene) Dekontaminationsmittelkonzentrationsabsenkungsmittel umfasst, um für Wartungszwecke oder nach Beendigung eines Produktionszyklusses die Dekontaminationsmittelkonzentration auch in der Dekontaminationskammer abbauen zu können. Bevorzugt ist es jedoch, auf derartige separate Absenkmittel zu verzichten und die Dekontaminationsmittelkonzentration bei geöffneten Verbindungs- bzw. Schleusentüren zur Beladeschleuse und/oder zur Entpackungsschleuse die Dekontaminationsmittelkonzentration über die jeweiligen Schleusen Dekontaminationsmittelabsenkmittel abzusenken.

Wie bereits angedeutet ist die Dekontaminationsvorrichtung bevorzugt derart betreibbar dass die Dekontaminationsmittelkonzentration während des Betriebs der Vorrichtung, d.h. auch während einer Be- und Entladung der Dekontaminationskammer in dieser auf einem hohen Niveau bleibt, ganz besonders bevorzugt sind hierzu die Beaufschlagungsmittel zum Beaufschlagen der Dekontaminationskammer mit Dekontaminationsmittel so angesteuert, dass eine, insbesondere überwachte bzw. gemessene Dekontaminationsmittelkonzentration in der Dekontaminationskammer auch während des Beladens und/oder Entladens der Dekontaminationskammer oberhalb eines Grenzwertes, insbesondere aus einem Wertebereich zwischen 300 ppm und 1200 ppm, ganz besonders zwischen 500 ppm und 1000 ppm gehalten wird bzw. haltbar ist. Jedenfalls ist es bevorzugt die Dekontaminationsmittelkonzentration, bis auf ein zeitweises Herstellen einer Verbindung zur Beladeschleuse und zur Entpackungsschleuse nicht aktiv über Absenkmittel abzusenken. Durch den Verzicht auf ein derartiges Absenken der Dekontaminationsmittelkonzentration in der Dekontaminationskammer und/oder das Halten der Dekontaminationskammer auf einem vorerwähnten Grenzwert kann die Verweilzeit der zu dekontaminierenden Verpackungsgüter in der Dekontaminationskammer verglichen mit dem Stand der Technik deutlich reduziert werden.

Die Erfindung betrifft ferner ein Isolatorsystem, insbesondere für pharmatechnische Anwendungen, umfassend einen Isolator, sowie eine an diesen derart angeschlossene, nach dem Konzept der Erfindung ausgebildete Dekontaminationsvorrichtung, dass aus dieser aus mit der Vorrichtung dekontaminierten Verbrauchsgüterumverpackungen entpackte, sterile bzw. vorsterilisierte Verbrauchsgüter, insbesondere Primärverpackungen dem Isolator, insbesondere unmittelbar, aus der Entpackungsschleuse zuführbar sind.

Darüber hinaus führt die Erfindung auf ein Verfahren zum Betreiben einer erfindungsgemäßen Dekontaminationsvorrichtung sowie eines erfindungsgemäßen Isolatorsystems. Erfindungsgemäß werden in der Dekontaminationsvorrichtung gleichzeitig eine Mehrzahl von (zeitlich beabstandet bzw. nacheinander, d.h. in unterschiedlichen Beladevorgängen der Dekontaminationskammer über die Beladeschleuse zugeführte, Verbrauchsgüterumverpackungen dekontaminiert und vom Einlass zum Auslass der Dekontaminationskammer transportiert. Die Beschickung der Dekontaminationskammer erfolgt dabei über die erwähnte Beladeschleuse, die der Dekontaminationskammer vorgeordnet ist und die mit zu dekontaminierenden Verbrauchsgüterumverpackungen beladen wird, wobei die Dekontaminationsmittelkonzentration vor der Beladung und nach Abkoppeln der Beladeschleuse von der Dekontaminationskammer abgesenkt wird. In der Entpackungsschleuse werden die vorsterilersten bzw. sterilen Verbrauchsgüter aus dem in der Dekontaminationskammer dekontaminierten Verbrauchsgüterumverpackungen entpackt, wobei die Dekontaminationsmittelkonzentration in der Entpackungsschleuse nach dem Abkoppeln der Entpackungsschleuse von der Dekontaminationskammer und vor dem Öffnen einer Verbindung zum Isolator abgesenkt wird.

Ganz besonders bevorzugt werden die aus unterschiedlichen Beladevorgängen der Dekontaminationskammer stammenden Verbrauchsgüterverpackungen in einer Reihe hintereinander durch die Dekontaminationskammer transportiert. Unabhängig von der Art und Weise des Transportes ist es wesentlich, dass gleichzeitig in der Dekontaminationskammer Verpackungsgüter aus unterschiedlichen Beladevorgängen der Beladeschleuse sowie Beschickungsvorgängen der Dekontaminationskammer aus der Beladeschleuse dekontaminiert und vom Einlass in Richtung Auslass transportiert werden.

Weitere Vorteile, Merkmale und Einzelheiten der Erfindung ergeben sich aus der nachfolgenden Beschreibung eines bevorzugten Ausführungsbeispiels sowie anhand der Zeichnungen.

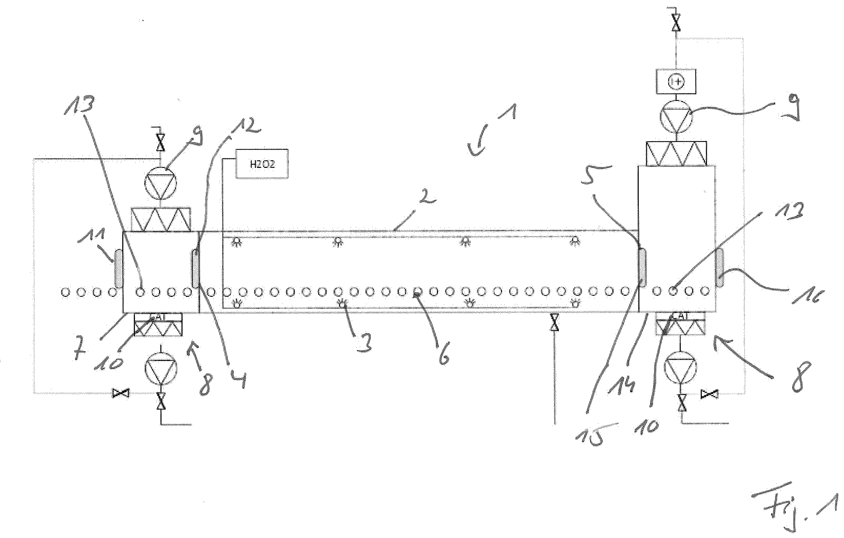

Diese zeigen in der einzigen
- Fig. 1: einen schematischen Aufbau einer bevorzugten Ausführungsform einer nach dem Konzept der Erfindung ausgebildeten Dekontaminationsvorrichtung.

In Fig. 1 ist eine nach dem Konzept der Erfindung ausgebildete Dekontaminationsvorrichtung 1 gezeigt. Diese umfasst eine langgestreckten, als Dekontaminationstunnel ausgebildete Dekontaminationskammer zwei mit Beaufschlagungsmitteln 3 zum Beaufschlagen von zu dekontaminierenden Verbrauchsgüterumverpackungen mit Dekontaminationsmittel. In dem vorliegenden Ausführungsbeispiel sind hierzu verschiedene Auslässe für Dekontaminationsmittel vorgesehen. Je nach Ausführung der Beaufschlagungsmittel können diese einen Verdampfer, insbesondere einen Blitzverdampfer zum Erzeugen von Dekontaminationsmitteldampf, insbesondere Wasserstoffperoxiddampf umfassen, der, bevorzugt über die Längserstreckung der Dekontaminationskammer 2 verteilt angeordneten Auslässen ausströmen kann. Bei einer alternativen Ausführungsform können Zerstäuber, insbesondere Ein- oder Mehrstoffdüsen zum Erzeugen eines Dekontaminationsmittelaerosols, insbesondere eines Wasserstoffperoxidaerosols in der Dekontaminationskammer 2 vorgesehen werden. Wesentlich ist jedenfalls, dass die zu dekontaminierenden Verbrauchsgüterumverpackungen während eines Transportes von einem Einlass 4 zu einem Auslass 5 der Dekontaminationskammer 2 mit Dekontaminationsmittel beaufschlagbar sind. Der Transport ist in dem gezeigten Ausführungsbeispiel über Transportmittel 6, hier beispielhaft in Form von einer Vielzahl von hintereinander angeordneten, rotierbar antreibbaren Transportrollen realisiert, wobei alternative Realisierungsformen, beispielsweise mit einem umlaufenden Band umsetzbar sind.

Der Dekontaminationskammer 2 ist eine Beladeschleuse 7 vorgeordnet, der Mittel 8 zum Absenken der Dekontaminationsmittelkonzentration zugeordnet sind. Diese Dekontaminationsmittelkonzentration rührt auf einer Leckage aus der Dekontaminationskammer 2 während eines Beschickungsvorgangs der Dekontaminationskammer 2 aus der Beladesschleuse 7 bei geöffneten Einlass 4, also bei an die Dekontaminationskammer 2 angekoppelter Beladeschleuse 7. Die Mittel 8 umfasen in dem gezeigten Ausführungsbeispiel mindestens ein Umluftgebläse 9 sowie einen Katalysator 10 zum Abbauen von Dekontaminationsmittel, wobei die Schleusenraumluft im Umluftbetrieb durch den Katalysator 10 zum Abbauen des Dekontaminationsmittels förderbar ist.

In dem gezeigten Ausführungsbeispiel umfasst die Beladeschleuse 7 eine Einlasstür 11 sowie eine Auslasstür 12, wobei die Auslasstür 12 gleichzeitig dazu dient, den Einlass 4 der Dekontaminationskammer 2 zu öffnen und damit die Beladeschleuse 7 anzukoppeln und wieder zu verschließen. Vor einem Beladevorgang der Beladeschleuse 7 mit einer Verbrauchsgüterumverpackung wird die Dekontaminationsmittelkonzentration über die Mittel 8 abgebaut, während die Einlasstür 11 und die Auslasstür 12 verschlossen sind. Daraufhin wird die Einlasstür 11 geöffnet und die Beladeschleuse 7 mit mindestens einer zu dekontaminierenden Verbrauchsgüterumverpackung beschickt. Daraufhin wird die Einlasstür 11 verschlossen und die Beladeschleuse 7 wird, vorliegend durch Öffnen der Auslasstür 12 bzw. des Einlasses 4 an die Dekontaminationskammer 2 angekoppelt und die mindestens eine Verbrauchsgüterumverpackung wird über Fördermittel 13 an die Transportmittel 6 übergeben, woraufhin der Einlass 4 bzw. die Auslasstür 12 wieder verschlossen und die Dekontaminationsmittelkonzentration in der Beladeschleuse 7 vor einem Öffnen der Einlasstür 11 für einen weiteren Beladevorgang abgebaut wird.

Dieser Vorgang wird immer wieder wiederholt, wobei sich dann in der Dekontaminationskammer 2 Verbrauchsgüterverpackungen aus mehreren Belade- bzw. Beschickungsvorgängen befinden, die dann gleichzeitig mittels der Transportmittel 6 in Richtung Auslass 5 transportiert und währenddessen dekontaminiert werden. Dabei ist die Länge bzw. der Förderweg der Verbrauchsgüterumverpackungen in der Dekontaminationskammer 2 so bemessen und auf die Geschwindigkeit der Transportmittel 6 bzw. die Transportgeschwindigkeit der Verbrauchsgüterumverpackungen abgestimmt, dass die Verbrauchsgüter jeweils eine Mindestzeit, von vorliegend bevorzugt zwischen 15 min und 25 min in der Dekontaminationskammer 2 verweilen, wovor sie über den Auslass 5 in eine Entpackungsschleuse 14 überführt werden. Der Auslass 5 ist offenbar und verschließbar mittels einer Einlasstür 15 der Entpackungsschleuse 14, über deren Auslasstür 16 der Isolator mittels in der Entpackungsschleuse 14 von nicht gezeigten Entpackungsmitteln aus einer zuvor dekontaminierten Verbrauchsgüterumverpackung entpackten Verbrauchsgütern, insbesondere Primärverpackungen versorgt wird. Bei geschlossenem Auslass 5 bzw. geschlossener Einlasstür 15 und geschlossener Auslasstür 16 wird die Dekontaminationsmittelkonzentration analog zur Beladeschleuse 7 über Mittel 8 abgesenkt. Auch diese Umfassen im Falle der Entpackungsschleuse 14 einen Katalysator 10 sowie mindestens ein Umluftgebläse 9 zum Erzeugen eines Umluftvolumenstroms durch den Katalysator 10 zum Abbau der Dekontaminationsmittelkonzentration. Zusätzlich oder alternativ können Frischluftfreispül- bzw. Versorgungsmittel vorgesehen werden.

Aus Fig. 1 ist weiter zu entnehmen, dass beiden Schleusen (Beladeschleuse 7 und Entpackungsschleuse 14) jeweils Luftfilter, insbesondere in Form von Hochleistungsschwebstofffiltern zugeordnet sind, durch die die Schleusenraumluft im Umluftbetrieb während des Förderns durch den Katalysator 10 gefördert wird bzw. förderbar ist.

In der Entpackungsschleuse 14 werden die dekontaminierten, aus der Dekontaminationskammer 2 bei geöffneten Auslass 5 kommenden Verbrauchsgüterumverpackungen von Fördermitteln 13 übernommen, die nach dem Entpacken der Verbrauchsgüter und dem Absenken der Dekontaminationsmittelkonzentration die Verbrauchsgüter über die Auslasstür 16 an einem nicht gezeigten Isolator überführen.

### Bezugszeichen

- 1: Dekontaminationsvorrichtung
- 2: Dekontaminationskammer
- 3: Beaufschlagungsmittel
- 4: Einlass der Dekontaminationskammer
- 5: Auslass der Dekontaminationskammer
- 6: Transportmittel der Dekontaminationskammer
- 7: Beladeschleuse
- 8: Mittel zum Absenken der Dekontaminationsmittelkonzentration
- 9: Umluftgebläse
- 10: Katalysator
- 11: Einlasstür der Beladeschleuse
- 12: Auslasstür der Beladeschleuse
- 13: Fördermittel
- 14: Entpackungsschleuse
- 15: Einlasstür der Entpackungsschleuse
- 16: Auslasstür der Entpackungsschleuse

## Patentansprüche

1. Dekontaminationsvorrichtung zum Dekontaminieren von Verbrauchsgüterumverpackungen mit darin befindlichen, bevorzugt als Primärverpackungen ausgebildeten, vorsterilisierten Verbrauchsgütern vor dem Zuführen der Verbrauchsgüter in einen Isolator, umfassend eine Dekontaminationskammer (2), aufweisend Beaufschlagungsmittel (3) zum Beaufschlagen der Verbrauchsgüterumverpackungen mit einem Dekontaminationsmittel, insbesondere einem Dekontaminationsmitteldampf oder -aerosol,
**dadurch gekennzeichnet,**
**dass** die Dekontaminationskammer (2) Transportmittel (6) zum gleichzeitigen Transportieren einer Mehrzahl von gleichzeitig zu dekontaminierenden Verbrauchsgüterumverpackungen während der Dekontamination von einem Einlass (4) zu einem Auslass (5) der Dekontaminationskammer (2) umfasst und dass der Dekontaminationskammer (2) eine Beladeschleuse (7) vorgeordnet ist, die mit zu dekontaminierenden Verbrauchsgüterumverpackungen beladbar ist, und dass der Dekontaminationskammer (2) eine Entpackungsschleuse (14) nachgeordnet ist, in der die sterilen Verbrauchsgüter vor dem Weitertransport in den Isolator aus der zuvor dekontaminierten Verbrauchsgüterumverpackung, insbesondere automatisiert, entpackbar sind, und dass der Beladeschleuse (7) und der Entpackungsschleuse (14) Mittel zum Absenken der Dekontaminationsmittelkonzentration (8) in der Beladeschleuse (7) bzw. der Entpackungsschleuse (14) in einem von der Dekontaminationskammer (2) abgekoppelten Betriebszustand zugeordnet sind.

2. Dekontaminationsvorrichtung nach Anspruch 1,
**dadurch gekennzeichnet,**
**dass** eine Baulänge der Dekontaminationskammer (2) so bemessen und auf eine Fördergeschwindigkeit der Transportmittel (6) abgestimmt ist, dass die Verbrauchsgüterumverpackungen auf ihrem Weg von Einlass (4) zum Auslass (5) der Dekontaminationskammer (2), bevorzugt bei einer kontinuierlichen oder intermittierenden Förderbewegung, mindestens 5 min, bevorzugt zwischen 10 min und 60min, weiter bevorzugt zwischen 10 min und 30 min, ganz besonders bevorzugt zwischen 15 min und 25 min in der mit Dekontaminationsmittel beaufschlagen Dekontaminationskammer (2) verweilen.

3. Dekontaminationsanordnung nach einem der Ansprüche,
**dadurch gekennzeichnet,**
**dass** ein von den Verbrauchsgüterumverpackung zwischen Einlass (4) und Auslass (5) zurücklegbarer Förderweg aus einem Wertebereich zwischen 1,0 m und 15,0 m, bevorzugt zwischen 2,0 m und 10,0 m, weiter bevorzugt zwischen 3,0 m und 8,0 m gewählt ist.

4. Dekontaminationsanordnung nach einem der vorhergehenden Ansprüche,
**dadurch gekennzeichnet,**
**dass** in der Beladeschleuse (7) und/oder der Entpackungsschleuse (14) Fördermittel (13) für die Verbrauchsgüterumverpackung vorgesehen sind.

5. Dekontaminationsanordnung nach einem der vorhergehenden Ansprüche,
**dadurch gekennzeichnet,**
**dass** die Beladeschleuse (7) und die Entpackungsschleuse (14) jeweils mindestens eine Einlasstür (11) als auch eine Auslasstür (12) aufweisen, wobei bevorzugt die Beladeschleuse (7) über deren Auslasstür (12) mit der Dekontaminationskammer (2) verbindbar und von dieser entkoppelbar ist und/oder die Entpackungsschleuse (14) über deren Einlasstür (15) mit der Dekontaminationskammer (2) verbindbar und von dieser entkoppelbar ist.

6. Dekontaminationsanordnung nach einem der vorhergehenden Ansprüche,
**dadurch gekennzeichnet,**
**dass** die Mittel zum Absenken der Dekontaminationsmittelkonzentration (8) Umlufterzeugungsmittel zum Erzeugen eines Umluftvolumenstroms durch Katalysatormittel zum Abbauen des Dekontaminationsmittel und/oder Frischluftversorgungsmittel zum Freispülen eines Raumvolumens mit, insbesondere steriler, Frischluft umfassen.

7. Dekontaminationsvorrichtung nach einem der vorhergehenden Ansprüche,
**dadurch gekennzeichnet,**
**dass** die Beaufschlagungsmittel (3) derart angesteuert sind, dass eine Dekontaminationsmittelkonzentration in den Dekontaminationskammern (2) auch während des Beladens und/oder Entladens der Dekontaminationskammern (2) oberhalb eines Grenzwertes, insbesondere aus einem Wertebereich zwischen 300 ppm und 1200 ppm gehalten wird und/oder dass auf ein aktives Absenken der Dekontaminationsmittelkonzentration in der Dekontaminationskammer (2) vor dem Beladen und/oder Entladen der Verbrauchsgüterumverpackungen in die bzw. aus der Dekontaminationskammer (2) verzichtet wird.

8. Isolatorsystem, insbesondere für pharmatechnische Anwendungen, umfassend einen Isolator sowie eine an diesen derart angeschlossene Dekontaminationsvorrichtung (1) nach einem der vorhergehenden Ansprüche, wobei die aus den dekontaminierten Verbrauchsgüterumverpackungen entpackten Verbrauchsgüter aus dieser, insbesondere unmittelbar aus der Entpackungsschleuse (14), dem Isolator, insbesondere einem Manipulatorraum des Isolators, steril zuführbar sind.

9. Verfahren zum Betreiben einer Dekontaminationsvorrichtung (1) nach einem der Ansprüche 1 bis 7 und/oder eines Isolatorsystems gemäß Anspruch 8, wobei die Verbrauchsgüterumverpackungen mit darin befindlichen, bevorzugt als Primärverpackungen ausgebildeten, vorsterilisierten Verbrauchsgütern in der Dekontaminationskammer (2) mit einem Dekontaminationsmittel, insbesondere einem Dekontaminationsmitteldampf oder -aerosol, beaufschlagt werden,
**dadurch gekennzeichnet,**
**dass** gleichzeitig eine Mehrzahl Verbrauchsgüterumverpackungen in der Dekontaminationskammer (2) dekontaminiert werden und während der Dekontamination mit den Transportmitteln (6) von dem Einlass (4) zu dem Auslass (5) der Dekontaminationskammer (2) transportiert werden, und dass die der Dekontaminationskammer (2) vorgeordnete Beladeschleuse (7) mit zu dekontaminierenden Verbrauchsgüterumverpackungen beladen wird, die aus der Beladeschleuse (7) der Dekontaminationskammer (2) zugeführt werden und dass in der der Dekontaminationskammer (2) nachgeordneten Entpackungsschleuse (14) die sterilen Verbrauchsgüter vor dem Weitertransport in den Isolator aus den zuvor dekontaminierten Verbrauchsgüterumverpackung, insbesondere automatisiert, entpackt werden und dass in der Beladeschleuse (7) vor dem Beladen mit einer Verbrauchsgüterumverpackung die Dekontaminationsmittelkonzentration abgesenkt wird und dass in der Entpackungsschleuse (14) vor dem Überführen der entpackten Verbrauchsgüter in den Isolator die Dekontaminationsmittelkonzentration abgesenkt wird.

10. Verfahren nach Anspruch 9,
**dadurch gekennzeichnet,**
**dass** die Verbrauchsgüterumverpackung auf ihrem Weg von Einlass (4) zum Auslass (5) der Dekontaminationskammer (2), bevorzugt im Rahmen einer einer kontinuierlichen oder intermittierenden Förderbewegung, mindestens 5 min, bevorzugt zwischen 10 min und 60min, weiter bevorzugt zwischen 10 min und 30 min, ganz besonders bevorzugt zwischen 15 min und 25 min in der mit Dekontaminationsmittel beaufschlagten Dekontaminationskammer (2) verweilen.

11. Verfahren nach einem der Ansprüche 9 oder 10,
**dadurch gekennzeichnet,**
**dass** die Verbrauchsgüterumverpackungen in Reihe hintereinander durch die Dekontaminationskammer (2) transportiert werden.

12. Verfahren nach einem der Ansprüche 9 bis 11,
**dadurch gekennzeichnet,**
**dass** die Dekontaminationsmittelkonzentration in den Dekontaminationsmittelkammern (2) auch während des Be- und Entladens der Dekontaminationsmittelkammern (2) oberhalb eines Grenzwertes, insbesondere zwischen 300 ppm und 1200 ppm gehalten wird und/oder und/oder dass auf ein aktives Absenken der Dekontaminationsmittelkonzentration in der Dekontaminationskammer (2) vor dem Beladen und/oder Entladen der Verbrauchsgüterumverpackungen in die bzw. aus der Dekontaminationskammer (2) verzichtet wird.
